# EUROPÄISCHE PATENTSCHRIFT

(11) **EP 1 091 923 B1**
(45) Veröffentlichungstag und Bekanntmachung des Hinweises auf die Patenterteilung: **26.03.2003**
(21) Anmeldenummer: 99931222.6
(22) Anmeldetag: 30.06.1999
(51) Int. Cl.: C07C 51/43, C07C 51/44, C07C 57/07

(54) **VERFAHREN ZUR REINIGUNG VON ACRYLSÄURE ODER METHACRYLSÄURE DURCH KRISTALLISATION UND DESTILLATION**
METHOD FOR PURIFYING ACRYLIC ACID OR METHACRYLIC ACID BY CRYSTALLIZATION AND DISTILLATION
PROCEDE POUR LA PURIFICATION D'ACIDE ACRYLIQUE OU D'ACIDE METHACRYLIQUE PAR CRISTALLISATION ET DISTILLATION

(30) Priorität: 01.07.1998 DE 19829477
(43) Veröffentlichungstag der Anmeldung: 18.04.2001
(73) Patentinhaber: BASF AKTIENGESELLSCHAFT, 67056 Ludwigshafen (DE)
(72) Erfinder: BASTIAENSEN, Erik, B-2950 Kapellen (BE); ECK, Bernd, D-68519 Viernheim (DE); THIEL, Joachim, D-67435 Neustadt (DE)
(74) Vertreter: Isenbruck, Günter, Dr.
(86) Internationale Anmeldenummer: EP9904516
(87) Internationale Veröffentlichungsnummer: WO00001657

(56) Entgegenhaltungen:
- EP-A- 0 616 998
- EP-A- 0 675 100
- EP-A- 0 792 867
- WO-A-98/01415

## Beschreibung

Die vorliegende Erfindung betrifft ein Verfahren zur Reinigung von Acrylsäure oder Methacrylsäure durch eine Kombination von Kristallisation und Destillation.

Acrylsäure ist eine bedeutende Grundchemikalie. Aufgrund ihrer sehr reaktionsfähigen Doppelbindung sowie der Säurefunktion eignet sie sich insbesondere als Monomeres zur Herstellung von Polymerisaten. z.B. für Klebstoffe, Dispersionen, Lacke oder "Superabsorber".

Es ist allgemein bekannt, daß Acrylsäure durch heterogen katalysierte Gasphasenoxidation von Propen mit molekularem Sauerstoff an im festen Aggregatzustand befindlichen Katalysatoren bei Temperaturen zwischen 200 und 400 °C zweistufig über Acrolein hergestellt werden kann. Hierbei werden oxidische Mehrkomponentenkatalysatoren z.B. auf Basis der Oxide der Elemente Molybdän, Bismut und Eisen (in der ersten Stufe) bzw. Molybdän und Vanadium (in der zweiten Stufe) eingesetzt.

In der DE-A-196 06 877 wird ein Verfahren zur Herstellung von Acrylsäure oder Methacrylsäure beschrieben, bei dem ein die Acrylsäure oder Methacrylsäure enthaltendes, durch katalytische Gasphasenoxidation hergestelltes Produktgemisch mit einem hochsiedenden Lösungsmittel absorbiert wird, das beladene Lösungsmittel durch Destillation in das Lösungsmittel und eine Rohsäure aufgetrennt wird und die Acrylsäure bzw. Methacrylsäure aus der Rohsäure durch Kristallisation gewonnen wird.

EP-A-0 616 998 betrifft ein Verfahren zur Reinigung von Acrylsäure mittels fraktionierter Kristallisation durch eine Kombination von dynamischer und statischer Kristallisation in mehreren Stufen, wobei der Rückstand der dynamischen Kristallisation durch die statische Kristallisation weitergereinigt wird und die dabei gewonnene Acrylsäure wieder der dynamischen Kristallisation zugeführt wird.

In EP-A-0 675 100 ist ein Verfahren zur Herstellung von α,β-ungesättigten C₃-C₆-Carbonsäuren und von Acrylsäure offenbart, bei dem die Säure durch oxidative Dehydrierung der entsprechenden gesättigten Säure hergestellt wird, das Dehydrierungsprodukt schmelzkristallisiert wird, die dabei erhaltene Mutterlauge fraktioniert destilliert wird und das Kopfprodukt der Destillation in den Dehydrierungsreaktor und das Sumpfprodukt der Destillation in die Schrnelzkristallisation rückgeführt bzw. das Kopfprodukt in die Schmelzkristallisation und das Sumpfprodukt in den Dehydrierungsreaktor rückgeführt werden. In einer weiteren Ausführungsform von EP-A-0 675 100 wird der bei der oxidativen Dehydrierung erhaltene Produktstrom zuerst fraktioniert destilliert, das Kopfprodukt der Destillation in den Dehydrierungsreaktor rückgeführt und das Sumpfprodukt der Destillation in eine Schmelzkristallisation geführt und die bei der Schmelzkristallisation erhaltene Mutterlauge anschließend in die Destillation rückgeführt.

Die Aufgabe der vorliegenden Erfindung bestand darin, ein Verfahren zu schaffen, bei dem Acrylsäure oder Methacrylsäure bei hoher Reinheit in hoher Ausbeute erhalten wird.

Es wird erfindungsgemäß vorgeschlagen, diese Aufgabe durch den kombinierten Einsatz von Kristallisation und Destillation zu lösen, wobei die bei einer ersten Kristallisation des Acrylsäure oder Methacrylsäure enthaltenden Gemisches entstehende Mutterlauge wenigstens zum Teil einer Destillation zugeführt wird, das dabei entstehende Kopfprodukt wenigstens zum Teil einer weiteren Kristallisation unterzogen wird und das sich hierbei bildende Kristallisat wiederum in die erste Kristallisation rückgeführt wird.

Somit betrifft die Erfindung ein Verfahren zur Reinigung von Acrylsäure oder Methacrylsäure durch Kristallisation und Destillation, das durch die folgenden Schritte gekennzeichnet ist:
(a) Kristallisation eines die Acrylsäure oder Methacrylsäure enthaltenden Gemisches unter Bildung eines an Acrylsäure oder Methacrylsäure angereicherten Produktkristallisats und einer Mutterlauge,
(b) Destillation wenigstens eines Teils der Mutterlauge aus Schritt (a) unter Bildung eines Destillationsrückstandes, der wenigstens teilweise abgezogen wird, und eines Kopfprodukts,
(c) Kristallisation wenigstens eines Teils des Kopfprodukts aus Schritt (b) unter Bildung eines Kristallisationsrückstandes, der abgezogen wird, und eines Kristallisats, und
(d) Rückführung des Kristallisats aus Schritt (c) in die Kristallisation (a).

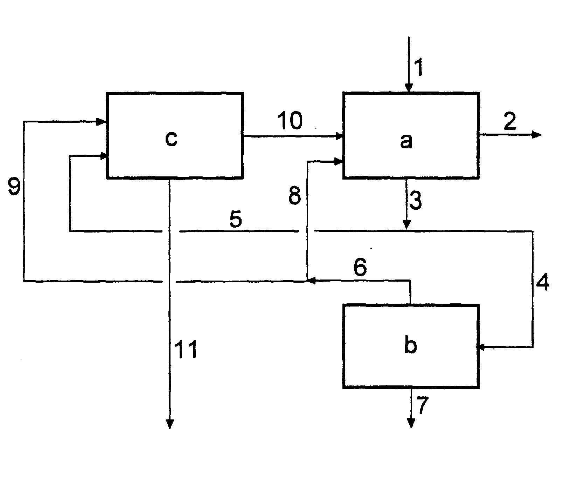

Bevorzugte Ausführungsformen der Erfindung sind in der nachfolgenden Beschreibung, den Unteransprüchen, der Figur und dem Beispiel definiert.

Die Figur zeigt ein bevorzugtes Ausführungsbeispiel zur Durchführung des erfindungsgemäßen Verfahrens.

Das erfindungsgemäß Acrylsäure oder Methacrylsäure enthaltene Gemisch unterliegt keinen besonderen Beschränkungen. Vorzugsweise werden Gemische verwendet, die 50 bis 99,95 Gew.-% Acrylsäure oder Methacrylsäure, vorzugsweise 60 bis 98 Gew.-% Acrylsäure oder Methacrylsäure, insbesondere 75 bis 95 Gew.-% Acrylsäure oder Methacrylsäure, jeweils bezogen auf 100 Gew.-% Gemisch, enthalten. Die Art der neben der Acrylsäure oder Methacrylsäure vorhandenen Verbindungen, Verunreinigungen oder Nebenkomponenten unterliegt keiner Beschränkung. Bevorzugt handelt es sich hierbei um bei der katalytischen Gasphasenoxidation zu Acrylsäure oder Methacrylsäure entstehende Verbindungen oder Nebenkomponenten, wie z.B. Wasser, Acrolein, Methacrolein, Essigsäure, Propionsäure, Formaldehyd oder andere Aldehyde. Daneben kann es sich auch um Lösungsmittel handeln, in die das bei der katalytischen Gasphasenoxidation zu Acrylsäure oder Methacrylsäure entstehende Produktgemisch absorbiert wird. Weiterhin kann das die Acrylsäure oder Methacrylsäure enthaltende Gemisch Polymerisationsinhibitoren oder Prozeßstabilisatoren als Nebenkomponenten enthalten. vorzugsweise handelt es sich hierbei um einen Stabilisator wie Phenothiazin oder einen anderen in EP-A-0 765 856 beschriebenen Stabilisator. Derartige Stabilisatoren werden der Acrylsäure oder Methacrylsäure nach ihrer Herstellung zugesetzt, um bei der Aufreinigung der Säure in den nachfolgenden Verfahrensschritten möglichst die Polymerisation der Säure zu inhibieren.

In einer bevorzugten Ausführungsform der Erfindung wird das die Acrylsäure oder Methacrylsäure enthaltende Gemisch hergestellt durch katalytische Gasphasenoxidation von C₃- bzw. C₄-Alkanen, -Alkenen, -Alkanolen und/oder - Alkanalen und/oder Vorstufen davon. Besonders vorteilhaft wird das Gemisch durch katalytische Gasphasenoxidation von Propen, Acrolein, tert.-Butanol, Isobuten, Isobutan, Isoburyraldehyd, Methacrolein, Isobuttersäure oder Methyltert.-butylether hergestellt. Als Ausgangsverbindungen können alle Vorstufen der genannten Verbindungen verwendet werden, bei denen sich die eigentliche C₃-/C₄-Ausgangsverbindung erst intermediär während der Gasphasenoxidation bildet. Beispielhaft genannt für die Herstellung der Methacrylsäure sei Methyl-tert.-butylether oder Isobuttersäure.
Besonders vorteilhaft ist die katalytische Gasphasenreaktion von Propen und/oder Acrolein zu Acrylsäure mit molekularem Sauerstoff nach bekannten Verfahren. insbesondere wie sie in den Druckschriften DE-A-1 962 431, DE-A-2 943 707, DE-PS 1 205 502, EP-A-0 257 565, EP-A-0 253 409, DE-AS 22 51 364, EP-A-0 117 146, GB-PS 1 450 986 und EP-A-0 293 224 beschrieben sind.

Vorzugsweise wird hierbei bei Temperaturen zwischen 200 und 450°C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch: katalytische Oxidehydrierung, wie z. B, in Catalysis Today 24 (1995), 307 - 313 oder US-A-5 510 558 beschrieben; durch homogene Oxidehydrierung, wie z. B. in CN-A-1 105 352 beschrieben; oder durch katalytische Dehydrierung, wie z. B. in EP-A-0 253 409, EP-A-0 293 224, DE-A-195 08 558 oder EP-A-0 117 146 beschrieben. Bei Einsatz eines Propen-/Propan-Gemischs wirkt Propan als Verdünnungsgas. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die o. g. mit Sauerstoff oder Luft oder einem Gemisch aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z.B. Luftstickstoff, einen oder mehrere gesättigte C₁-C₆-Kohlenwasserstoffe, insbesondere Methan und/oder Propan und/oder Wasserdampf enthält, kann daher nur einen kleinen Teil der Reaktionswärme aufnehmen. Obwohl die Art der verwendeten Reaktoren an sich keiner Beschränkung unterliegt, werden meist Rohrbündelwärmetauscher verwendet, die mit dem Oxidationskatalysator gefüllt sind, da bei diesen der überwiegende Teil der bei der Reaktion freiwerdenden Wärme durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propion säure, Formaldehyd, weitere Aldehyde und Maleinsäureanhydrid enthalten kann. Üblicherweise enthält das Reaklionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbindungen mit molekularern Sauerstoff hergestellt werden. Besonders vorteilhaft ist die Methacrylsäure, z. B. durch katalytische Gasphasenoxidation von Isobuten, Isobutan. tert.-Butanol, Isobutyraldehyd, Methacrolein oder Methyl-tert.-butylether erhältlich. Als Katalysatoren werden ebenfalls übergangsmetallische Mischoxidkatalysatoren (z. B. Mo, V, W und/oder Fe) verwendet. Besonders geeignete Verfahren sind solche, bei denen die Herstellung ausgehend von Methacrolein erfolgt, insbesondere dann, wenn das Methacrolein durch gasphasenkatalytische Oxidation von fett.-Butanol, Isobutan oder Isobuten oder durch Umsetzung von Formaldehyd mit Propionaldehyd gemäß EP-B-0 092 097 oder EP-B-0 058 927 erzeugt wird. Somit besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrylsäure und (2) anschließende Oxidation des Methacroleins zu Methacrylsäure.

Ebenso wie bei der Herstellung der Acrylsäure wird nicht reine Methacrolein, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann. Das erfindungsgemäße Verfahren wird insbesondere dann eingesetzt, wenn das Reaktionsgemisch 0,02 bis 2 Gew.-% Methacrolein bezogen auf das gesamte Reaktionsgemisch und ansonsten im wesentlichen die gleichen entsprechenden Bestandteile wie bei der Herstellung der Acrylsäure enthält.
In einer bevorzugten Ausführungsform wird als das die Acrylsäure oder Methacrylsäure enthaltende Gemisch eine Rohsäure verwendet, wie sie durch die oben genannte Herstellung durch katalytische Gasphasenoxidation sowie gemäß DE-A-196 06 877 durch anschließende Absorption mit einem Lösungsmittel und Destillation erhalten wird.

Das erfindungsgemäße Verfahren wird insbesondere angewendet, wenn die zu reinigende Säure neben den oben genannten Verunreinigungen zwischen 50 ppm und 2 Gew.-% Maleinsäure und/oder Maleinsäureanhydrid enthält.

Das für die Kristallisation (a) und für die Kristallisation (c) verwendete Kristallisationsverfahren unterliegt keiner Beschränkung. Es kann kontinuierlich oder diskontinuierlich, einstufig oder mehrstufig durchgeführt werden. In einer bevorzugten Ausführungsform wird eine der beiden Kristallisationen (a) oder (c) oder es werden beide als fraktionierte (mehrstufige) Kristallisation durchgeführt. Üblicherweise werden bei fraktionierter Kristallisation alle Stufen, die ein Kristallisat erzeugen, das reiner ist als das zugeführte, die Acrylsäure oder Methacrylsäure enthaltende Gemisch, Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugerührt wird. In einer bevorzugten Ausgestaltung der Erfindung erfolgt die Kristallisation in Apparaten, in denen die Kristalle im Kristallisationsapparat an gekühlten Flächen aufwachsen, d.h. im Apparat fixiert sind (z.B. dynamisches Schichtkristallverfahren der Firma Sulzer Chemtech oder statisches Kristallisationsverfahren der Firma BEFS PROKEM). Die Kristallisation kann dynamisch und/oder statisch durchgeführt werden, wobei eine Kombination von dynamischer und statischer Kristallisation besonders bevorzugt ist. Bei letzterer Ausführungsform wird, wie in EP-A-0 616 998 beschrieben, vorzugsweise der Rückstand der dynamischen Kristallisation der statischen Kristallisation zugeführt und das Kristallisat der statischen Kristallisation der dynamischen Kristallisation zugeführt. Die Art und Weise der Durchführung der dynamischen und/oder statischen Kristallisation ist hierbei nicht kritisch, Bei der statischen Kristallisation wird die flüssige Phase nur durch freie Konvektion bewegt, während bei der dynamischen Kristallisation die flüssige Phase durch erzwungene Konvektion bewegt wird. Letzteres kann durch eine erzwungene Strömung in voll durchströmten Apparaten (vgl. z.B. DE-OS- 2 606 364) oder durch die Aufgabe eines Riesel- oder Fallfilms auf eine gekühlte Wand (vgl. z.B. DT 1 769 123 und EP-A-0 218 545) erfolgen. Vorteilhafterweise wird die statische Kristallisation in den Abtriebsstufen eingesetzt, und zwar dann, wenn die Ausbeute an der Säure noch weiter erhöht werden soll.

In einer besonders bevorzugten Ausgestaltung der Erfindung wird die Kristallisation (a) als dynamische Kristallisation durchgeführt und die Kristallisation (c) als statische Kristallisation durchgeführt.

In einer vorteilhaften Ausgestaltung der Erfindung erfolgt die Kristallisation durch Kühlung von Apparatewänden oder durch Verdampfung der Lösung im Vakuum. Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet.

Daneben besteht auch die Möglichkeit, die Wärme über die Wand eines Rührkessels mit wandgängigem Rührer abzuführen. Eine weitere bevorzugte Ausrührungsform bei der Kühlungskristallisation ist die Verwendung von Kühlscheibenkristallisatoren, wie sie z.B. von der Firma Gouda (Holland) hergestellt werden. Bei einer weiteren geeigneten Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparates wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um, usw. Diese Variante kann auch mit mehr als zwei Apparaten im Wechsel betrieben werden. Außerdem kann die Kristallisation durch eine herkömmliche Verdampfung der Lösung im Vakuum erfolgen.

Vorteilhafterweise liegt die Temperatur der Lösung während der Kristallisation (a) zwischen -15 und +14 °C, insbesondere zwischen 0 und +14 °C, während die Temperatur bei der Kristallisation (c) zwischen -30 und +5 °C, insbesondere zwischen -15 und 0 °C liegt. Der Feststoffgehalt im Kristallisator liegt bei beiden Kristallisationen (a) und (c) vorteilhafterweise zwischen 0 und 85 g, bevorzugt zwischen 25 und 80 g Feststoff/100 g.

Die Art der Abtrennung der nach der Kristallisation (a) und (c) erhaltenen Acrylsäurekristalle oder Methacrylsäurekristalle von der Mutterlauge unterliegt keinen besonderen Beschränkungen. Für den Fall der Schichtkristallisation oder der Statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. Vorzugsweise werden die Kristalle durch Filtrieren und/oder Zentrifugieren von der Mutterlauge abgetrennt. Vorteilharterweise wird dem Filtrieren oder Zentrifugieren eine Voreindikung der Suspension, z.B. durch Hydrozyklone, vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafteriveise mittels Filternutschen, die kontinuierlich oder diskontinuierlich, mit oder ohne Rührwerk, oder mittels Bandfilter betrieben werden. Allgemein kann das Filtrieren unter Druck oder im Vakuum erfolgen. Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Die verwendete Waschflüssigkeit unterliegt hierbei keiner Einschränkung. Vorteilhafterweise wird jedoch mit Reinprodukt gewaschen, d.h. mit einer Flüssigkeit, die Acrylsäure oder Methacrylsäure enthält, deren Reinheit höher ist als die des zu waschenden Kristallkuchens. Daneben ist auch eine Wäsche mit Wasser möglich. Das Waschen kann in hierfür üblichen Apparaten erfolgen, wie Waschkolonnen, in denen die Abtrennung der Mutterlauge und das Waschen in einem Apparat erfolgen, in Zentrifugen, die ein- oder mehrstufig betrieben werden können, oder in Filternutschen oder Bandfiltern. Das Waschen kann auf Zentrifugen oder Bandfiltern ein- oder mehrstufig durchgeführt werden, wobei die Waschflüssigkeit im Gegenstrom zum Kristallkuchen geführt werden kann. Daneben kann ein sogenanntes Schwitzen zur Erhöhung der Reinheit der Kristalle vorgesehen sein, bei dem es sich um ein lokales Abschmelzen verunreinigter Bereiche handelt. Besonders bevorzugt ist die Durchführung des Schwitzens auf Zentrifugen oder Bandfiltern, jedoch kann auch die Durchführung einer Kombination aus Waschen und Schwitzen in einem Apparat geeignet sein.

Wird die Kristallisation (a) mehrstufig durchgeführt, so wird vorzugsweise die Mutterlauge mit der niedrigsten Reinheit der Destillation (b) zugeführt. Daneben besteht auch die Möglichkeit, die Mutterlaugen aller oder eines Teils der Kristallisationsstufen von Kristallisation (a) zu kombinieren und dann der Destillation (b) zuzuführen. Wird die Kristallisation (c) mehrstufig durchgeführt, so wird vorzugsweise der Kristallisationsrückstand mit der niedrigsten Reinheit abgeführt und das Kristallisat mit der höchsten Reinheit der Kristallisation (a) zugeführt.

Die Menge an Mutterlauge aus Schritt (a), die der Destillation (b) zugeführt wird, beträgt vorzugsweise 5 bis 100 Gew.-%, insbesondere 50 bis 100 Gew.-%, am meisten bevorzugt 90 bis 100 Gew.-%. Nicht der Destillation (b) zugeführte Mutterlauge aus Schritt (a) wird zweckmäßigerweise der Kristallisation (c) zugeführt.

Für die Destillation (b) kann grundsätzlich jede Destillationskolonne verwendet werden. Die Destillation, Destillationsstufe oder der Destillationsschritt (b) kann einstufig oder mehrstufig durchgeführt werden. Bei einer mehrstufigen oder fraktionierten Destillation (auch Rektifikation genannt), die vorteilhafterweise in Rektifizierkolonnen durchgeführt wird, wird eine Kolonne mit Siebböden, z.B. Dualflowböden oder Querstromsiebböden aus Metall, verwendet. Die Destillation kann auch mittels eines Verdampfers und eines nachgeschalteten Kondensators durchgeführt werden. Besonders bevorzugt sind hierbei Dünnschichtverdampfer als Fallstromverdampfer oder Dünnschichtverdampfer mit rotierenden Wischern. Als Kondensatoren werden die üblichen Kondensatoren eingesetzt, wobei diese keinen Beschränkungen unterliegen. Bei einer einstufigen Destillation wird zweckmäßigerweise ein einfacher Verdampfer, z.B. eine Blase, und ein üblicher Kondensator eingesetzt.

Der Acrylsäure kann zu ihrer Stabilisierung und zur Verhinderung einer Polymerisation während der Destillation (b) ein Stabilisator wie Phenothiazin oder ein anderer in z.B. EP-A-0 765 856 offenbarter Stabilisator zugegeben werden, sofern ein solcher noch nicht oder nicht in ausreichender Menge vorhanden ist.

Die Menge an Kopfprodukt aus der Destillation (b), die der Kristallisation (c) zugeführt wird, beträgt vorzugsweise 5 bis 100 Gew.-%, insbesondere 50 bis 100 Gew.-%, am meisten bevorzugt 80 bis 100 Gew.-% Kopfprodukt. Nicht in die Kristallisation (c) zugeführtes Kopfprodukt wird vorteilhafterweise der Kristallisation (a) zugeführt. Wenigstens ein Teil des Destillationsrückstandes, vorzugsweise 5 bis 100 Gew.-%, insbesondere 5 bis 50 Gew.-%, des Destillationsrückstandes aus der Destillation (b) wird aus dem Verfahren ausgeschleust, während 0 bis 95 Gew.-%, insbesondere 50 bis 95 Gew.-%, des Destillationsrückstandes in die Herstellung der Acrylsäure oder Methacrylsäure rückgeführt wird, vorzugsweise in die katalytische Gasphasenoxidation zur Herstellung der Acrylsäure oder Methacrylsäure, wie sie oben beschrieben worden ist.

Die Erfindung bietet den Vorteil, daß bei hoher Reinheit der gewünschten Säure gleichzeitig eine hohe Ausbeute an der Säure erreicht wird. Anders als in EP-A-0 675 100 werden erfindungsgemäß zwei Kristallisationen durchgeführt, die dadurch miteinander verknüpft sind, daß das Kristallisat der zweiten Kristallisation der ersten Kristallisation zugeführt wird, so daß die zweite Kristallisation als eine Art "Abtriebsstufe" der ersten Kristallisation angesehen werden kann. Gleichzeitig sieht die Erfindung das teilweise Abziehen sowohl des Destillationsrückstandes als auch das Abziehen eines Kristallisationsrückstandes aus dem Verfahren vor.

Demgegenüber offenbart EP-A-0 675 100 lediglich eine Kristallisation, die ggf. mehrstufig mit Reinigungsstufen durchgeführt werden kann, sowie das Abziehen von Rückstand aus dem Prozeß an nur einer Stelle, nämlich entweder aus der Kristallisation oder aus der Destillation. Somit bietet die Erfindung gegenüber EP-A-0 675 100 den Vorteil, daß aus dem Prozeß destillativ und kristallisativ schwer abtrennbare Stoffe gleichzeitig abgezogen werden können. Beispielsweise wird Propionsäure, die destillativ schwer oder nicht abtrennbar ist, über den Kristallisationsrückstand abgezogen, während Stoffe, die in der Kristallisationsstufe leicht zusammen mit der gewünschten Säure ausfallen (wie z.B. Phenothiazin, Maleinsäure) und dadurch die Trennwirkung der Kristallisation herabsetzen würden, vor Überschreiten ihrer Löslichkeitsgrenze durch Ausschleusen des Destillationsrückstandes aus dem Prozeß abgerührt werden.
Die Figur beschreibt eine bevorzugte Ausführungsform des erfindungsgemäßen Verfahrens. Über Zuleitung 1 wird das die Acrylsäure oder Methacrylsäure enthaltende Gemisch der Kristallisation (a) zugeführt. Nach Durchführung der Kristallisation (a) und einer (nicht gezeigten) Fest-Flüssig-Abtrennung erhält man ein Produktkristallisat, das über Leitung 2 abgezogen wird, sowie eine Mutterlauge, die über Leitung 3 wenigstens zum Teil über Leitung 4 der Destillation (b) zugeführt wird. Nicht der Destillation (b) zugeführte Mutterlauge aus Leitung 3 wird über Leitung 5 der Kristallisation (c) zugeführt. Bei der Destillation (b) wird ein Destillationsrückstand erhalten, der über Leitung 7 aus dem Prozeß abgezogen wird, sowie ein Kopfprodukt, das über Leitung 6 der Destillation entnommen wird. Wenigstens ein Teil des Kopfprodukts aus Leitung 6 wird über Leitung 9 der Kristallisation (c) zugeführt. Nicht der Kristallisation (c) zugeführtes Kopfprodukt aus Leitung 6 wird über Leitung 8 der Kristallisation (a) zugeführt. Nach Durchrührung der Kristallisation (c) und einer (nicht gezeigten) Fest-Flüssig-Abtrennung erhält man ein Kristallisat, das über Leitung 10 der Kristallisation (a) zugeführt wird, und einen Kristallisationsrückstand, der über Leitung 11 aus dem Prozeß abgeführt wird.

Die Erfindung wird anhand des folgenden Beispiels, das eine bevorzugte Ausrührungsform der Erfindung darstellt, näher erläutert.

### BEISPIEL

Ein Strom gemäß der unten in Tabelle 1 angegebenen Ausgangszusammensetzung wurde einer fünfstufigen Kristallisation (Kristallisation (a)) mit zwei Reinigungsstufen und drei Abtriebsstufen unterzogen, wobei alle Stufen dynamisch durchgeführt wurden. Der Feststoffgehalt im Kristallisator betrug jeweils 72 g/100 g. Nach der Fest-Flüssig-Abtrennung wurden in der 2. Reinigungsstufe (d.h. der Reinigungsstufe höchster Reinheit) ein Produktkristallisat und in der 3. Abtriebsstufe (d.h. der Abtriebsstufe niedrigster Reinheit) eine Mutterlauge gemäß folgender Tabelle 1 erhalten.

**Tabelle 1**

| **Ströme** | **Ausgangszusammensetzung** | **Produktkristallisat** | **Mutterlauge** |
|---|---|---|---|
| Acrylsäure | 99,48 Gew.-% | 99,94 | 77,63 Gew.-% |
| Essigsäure | 2600 ppm | 374 ppm | 13,285 Gew.-% |
| Propionsäure | 358 ppm | 99 ppm | 1,348 Gew.-% |
| Wasser | 460 ppm | 91 ppm | 2,002 Gew.-% |
| Phenothiazin | 420 ppm | 1 ppm | 1,192 Gew.-% |
| Furan-II-aldehyd | 335 ppm | 0,5 ppm | 1,307 Gew.-% |
| Sonstige | 0,1027 Gew.-% | 35 ppm | 3,236 Gew.-% |

Die Mutterlauge gemäß Tabelle 1 wurde zu 100 % einer Destillation (b) zugeführt. Diese Destillation wurde einstufig in einem Dünnschichtverdampfer durchgeführt. Der hierbei entstehende Brüden wurde nach üblichen Verfahren kondensiert. Das Verhältnis von Kopfprodukt zu Mutterlauge im Verdampfer betrug 80g/100 g. Die folgende Tabelle 2 gibt die Zusammensetzung des bei der Destillation (b) erhaltenen Kopfprodukts sowie Destillationsrückstandes an.

**Tabelle 2**

| **Ströme** | **Kopfprodukt** | **Rückstand** |
|---|---|---|
| Acrylsäure | 77,74 Gew.-% | 77,17 Gew.-% |
| Essigsäure | 15,41 Gew.-% | 4,783 Gew.-% |
| Propionsäure | 1,335 Gew.-% | 1,402 Gew.-% |
| Wasser | 2,382 Gew.-% | 0,481 Gew.-% |
| Phenothiazin | 0,107 Gew.-% | 5,530 Gew.-% |
| Furan-II-aldehyd | 1,137 Gew.-% | 1,9864 Gew.-% |
| Sonstige | 1,889 Gew.-% | 8,648 Gew.-% |

Der Rückstand aus der Destillation (b) wurde verworfen, während das Kopfprodukt zu 100 % einer Kristallisation (c) zugeführt wurde. Diese Kristallisation (c) wurde in zwei, jeweils statischen Stufen (d.h. eine Reinigungsstufe und eine Abtriebsstute) durchgeführt. Der Feststoffgehalt betrug jeweils 50 g/100 g. Hierbei wurde folgendes Kristallisat in der Reinigungsstufe und folgender Rückstand in der Abtriebsstufe erhalten:

**Tabelle 3**

| **Ströme** | **Kristallisat** | **Rückstand** |
|---|---|---|
| Acrylsäure | 83,28 Gew.-% | 61.12 Gew.-% |
| Essigsäure | 12.134 Gew.-% | 25,24 Gew.-% |
| Propionsäure | 1,079 Gew.-% | 2,103 Gew.-% |
| Wasser | 1.674 Gew.-% | 4,507 Gew.-% |
| Phenothiazin | 0,091 Gew.-% | 1,146 Gew.-% |
| Furan-II-aldehyd | 0,677 Gew.-% | 2,518 Gew.- % |
| Sonstige | 1,065 Gew.-% | 3,366 Gew.-% |

Das Kristallisat gemäß Tabelle 3 wurde zu 100% der Kristallisationsstufe (a) zugeführt, während der Rückstand gemäß Tabelle 3 verworfen wurde.

Wie insbesondere Tabelle 1 des Beispiels zeigt, läßt sich mit dem erfindungsgemäßen Verfahren eine Acrylsäure relativ hoher Reinheit in - bedingt durch die Rückführung - hoher Ausbeute herstellen.

## Patentansprüche

1. Verfahren zur Reinigung von Acrylsäure oder Methacrylsäure durch Kristallisation und Destillation, **gekennzeichnet durch** die folgenden Schritte:
(a) Kristallisation eines die Acrylsäure oder Methacrylsäure enthaltenden Gemisches unter Bildung eines an Acrylsäure oder Methacrylsäure angereicherten Produktkristallisats und einer Mutterlauge,
(b) Destillation wenigstens eines Teils der Mutterlauge aus Schritt (a) unter Bildung eines Destillationsrückstandes, der wenigstens teilweise abgezogen wird, und eines Kopfprodukts,
(c) Kristallisation wenigstens eines Teils des Kopfprodukts aus Schritt (b) unter Bildung eines Kristallisationsrückstandes, der abgezogen wird, und eines Kristallisats, und
(d) Rückführung des Kristallisats aus Schritt (c) in die Kristallisation (a).

2. Verfahren nach Anspruch 1, **dadurch gekennzeichnet, daß** das die Acrylsäure oder Methacrylsäure enthaltende Gemisch 50 bis 99,95 Gew.-% Acrylsäure oder Methacrylsäure bezogen auf das Gemisch enthält.

3. Verfahren nach Anspruch 1 oder 2, **dadurch gekennzeichnet, daß** die Kristallisation (a) und die Kristallisation (c) jeweils ein- oder mehrstufig durchgeführt werden.

4. Verfahren nach einem der Ansprüche 1 bis 3, **dadurch gekennzeichnet, daß** 5 bis 100 Gew.-% der Mutterlauge aus Schritt (a) in Schritt (b) destilliert werden.

5. Verfahren nach einem der vorhergehenden Ansprüche, **dadurch gekennzeichnet, daß** die Destillation (b) einstufig oder mehrstufig (fraktioniert) durchgeführt wird.

6. Verfahren nach einem der vorhergehenden Ansprüche, **dadurch gekennzeichnet, daß** 5 bis 100 Gew.-% des Kopfprodukts aus Schritt (b) in Schritt (c) kristallisiert werden.

7. Verfahren nach einem der vorhergehenden Ansprüche, **dadurch gekennzeichnet, daß** die Kristallisation (a) als dynamische Kristallisation und die Kristallisation (c) als statische Kristallisation durchgeführt werden.

8. Verfahren nach einem der Ansprüche 1 bis 6, **dadurch gekennzeichnet, daß** als Kristallisation (a) und/oder Kristallisation (c) eine kombinierte dynamische und statische Kristallisation durchgeführt wird, wobei der Rückstand der dynamischen Kristallisation der statischen Kristallisation zugeführt wird und das Kristallisat der statischen Kristallisation der dynamischen Kristallisation zugeführt wird.

9. Verfahren nach einem der vorhergehenden Ansprüche, **dadurch gekennzeichnet, daß** das die Acrylsäure oder Methacrylsäure enthaltende Gemisch durch katalytische Gasphasenoxidation von C₃-/C₄-Alkanen, -Alkenen, -Alkanolen und/oder -Alkanalen und/oder Vorstufen davon hergestellt wird.

10. Verfahren nach einem der vorhergehenden Ansprüche, **dadurch gekennzeichnet, daß** 0 bis 95 Gew.-% des Rückstandes der Destillation (b) in das Verfahren zur Herstellung der Acrylsäure oder Methacrylsäure rückgeführt werden.

## Claims

1. A process for the purification of acrylic acid or methacrylic acid by crystallization and distillation, which comprises the following steps:
(a) crystallization of a mixture containing the acrylic acid or methacrylic acid with formation of product crystals having a higher concentration of acrylic acid or methacrylic acid and of a mother liquor,
(b) distillation of at least part of the mother liquor from step (a) with formation of a distillation residue, which is at least partly removed, and of a top product,
(c) crystallization of at least part of the top product from step (b) with formation of a crystallization residue, which is removed, and of crystals and
(d) recycling of the crystals from step (c) to the crystallization (a).

2. A process as claimed in claim 1, wherein the mixture containing the acrylic acid or methacrylic acid contains from 50 to 99.95% by weight, based on the mixture, of acrylic acid or methacrylic acid.

3. A process as claimed in claim 1 or 2, wherein the crystallization (a) and the crystallization (c) are each carried out in one stage or a plurality of stages.

4. A process as claimed in any of claims 1 to 3, wherein from 5 to 100% by weight of the mother liquor from step (a) are distilled in step (b).

5. A process as claimed in any of the preceding claims, wherein the distillation (b) is carried out in one stage or a plurality of stages (as a fractional distillation).

6. A process as claimed in any of the preceding claims, wherein from 5 to 100% by weight of the top product from step (b) are crystallized in step (c).

7. A process as claimed in any of the preceding claims, wherein the crystallization (a) is carried out as a dynamic crystallization and the crystallization (c) as a static crystallization.

8. A process as claimed in any of claims 1 to 6, wherein a combined dynamic and static crystallization is carried out as crystallization (a) and/or crystallization (c), the residue of the dynamic crystallization being fed to the static crystallization and the crystals of the static crystallization being fed to the dynamic crystallization.

9. A process as claimed in any of the preceding claims, wherein the mixture containing the acrylic acid or methacrylic acid is prepared by catalytic gas-phase oxidation of C₃-/C₄-alkanes, -alkenes, -alkanols and/or -alkanals and/or intermediates thereof.

10. A process as claimed in any of the preceding claims, wherein from 0 to 95% by weight of the residue of the distillation (b) are recycled to the process for the preparation of the acrylic acid or methacrylic acid.

## Revendications

1. Procédé de purification d'acide acrylique ou d'acide méthacrylique par cristallisation et distillation, **caractérisé par** les étapes suivantes:
a) Cristallisation d'un mélange contenant de l'acide acrylique ou de l'acide méthacrylique, avec formation d'un produit cristallisé enrichi en acide acrylique ou en acide méthacrylique et d'une eau mère,
b) Distillation d'au moins une partie de l'eau mère obtenue à l'étape (a) avec formation d'un résidu de distillation, qui est au moins en partie soutiré, et d'un produit de tête,
c) Cristallisation d'au moins une partie du produit de tête de l'étape (b) avec formation d'un résidu de cristallisation, qui est soutiré, et d'un produit cristallisé, et
d) Recyclage du produit cristallisé de l'étape (c) dans la cristallisation (a).

2. Procédé selon la revendication 1, **caractérisé en ce que** le mélange contenant de l'acide acrylique ou de l'acide méthacrylique contient de 50 à 99,95% en poids d'acide acrylique ou d'acide méthacrylique, par rapport au mélange.

3. Procédé selon la revendication 1 ou 2, **caractérisé en ce que** la cristallisation (a) et la cristallisation (c) sont à chaque fois entreprises en une ou plusieurs étapes.

4. Procédé selon l'une des revendications 1 à 3, **caractérisé en ce que** l'on distille dans l'étape (b) de 5 à 100% en poids de l'eau mère de l'étape (a).

5. Procédé selon l'une des revendications qui précèdent, **caractérisé en ce que** la distillation (b) est entreprise en une étape ou en plusieurs étapes (fractionnée).

6. Procédé selon l'une des revendications qui précèdent, **caractérisé en ce que** l'on cristallise dans l'étape (c) de 5 à 100% en poids du produit de tête de l'étape (b).

7. Procédé selon l'une des revendications qui précèdent, **caractérisé en ce que** la cristallisation (a) est entreprise en tant que cristallisation dynamique et la cristallisation (c) en tant que cristallisation statique.

8. Procédé selon l'une des revendications 1 à 6, **caractérisé en ce que** la cristallisation (a) et / ou la cristallisation (c) sont entreprises en cristallisation dynamique et statique combinée, le résidu de la cristallisation dynamique étant envoyé à la cristallisation statique et le cristallisat de la cristallisation statique étant envoyé à la cristallisation dynamique.

9. Procédé selon l'une des revendications qui précèdent, **caractérisé en ce que** le mélange contenant de l'acide acrylique ou de l'acide méthacrylique est préparé par oxydation catalytique en phase gazeuse d'alcanes, alcènes, alcanols et / ou alcanals en C₃ / C₄ et / ou de progéniteurs de ceux-ci.

10. Procédé selon l'une des revendications qui précèdent, **caractérisé en ce que** de 0 à 95% en poids du résidu de la distillation (b) sont recyclés dans le procédé de préparation de l'acide acrylique ou de l'acide méthacrylique.
